# EUROPEAN PATENT SPECIFICATION

(11) **EP 2 328 912 B1**
(45) Date of publication and mention of the grant of the patent: **08.04.2015**
(21) Application number: 09752003.5
(22) Date of filing: 27.09.2009
(51) Int. Cl.: C07K 14/15, G01N 33/569

(54) **Method for producing purified bovine leukemia virus surface antigen gp51**
Verfahren zur Herstellung von gereinigtem Rinder-Leukämie-Virus Oberflächenantigen gp51
Procédé de production d'antigène de surface gp51 du virus de la leucémie bovine purifié

(30) Priority: 29.09.2008 PL 38617608
(43) Date of publication of application: 08.06.2011
(73) Proprietor: Instytut Immunologii I Terapii Doswiadczalnej Pan, 53-114 Wroclaw (PL)
(72) Inventor: RAPAK, Andrzej, 49-306 Brzeg (PL); ZIOLO, Ewa, 50-550 Wroclaw (PL)
(74) Representative: Witek, Rafal
(86) International application number: PCT/PL2009/050027
(87) International publication number: WO 2010/036134

(56) References cited:
- DE GIUSEPPE ANTONIO ET AL: "Expression of the bovine leukemia virus envelope glycoprotein (gp51) by recombinant baculovirus and its use in an enzyme-linked immunosorbent assay." CLINICAL AND DIAGNOSTIC LABORATORY IMMUNOLOGY JAN 2004, vol. 11, no. 1, January 2004 (2004-01), pages 147-151, XP002563707 ISSN: 1071-412X
- MERZA M ET AL: "Immunoaffenity purification of two major proteins of bovine leukemia virus (gp51 and p24) and their use for discrimination between vaccinated and infected animals" JOURNAL OF VIROLOGICAL METHODS, ELSEVIER BV, NL, vol. 33, no. 3, 1 August 1991 (1991-08-01) , pages 345-353, XP023794780 ISSN: 0166-0934 [retrieved on 1991-08-01]
- CAMARGOS M ET AL.: "Evaluation of diagnostic tests to bovine leukemia virus" REVISTA PORTUGUESA DE CIENCIAS VERTINARIAS, vol. 102, no. 561-562, 2007, pages 169-173, XP002563709
- MAGER A ET AL: "T cell proliferative response to bovine leukaemia virus (BLV): identification of T cell epitopes on the major core protein (p24) in BLV-infected cattle with normal haematological values." THE JOURNAL OF GENERAL VIROLOGY SEP 1994, vol. 75 ( Pt 9), September 1994 (1994-09), pages 2223-2231, XP002563708 ISSN: 0022-1317
- BEIER D ET AL: "Establishment of a new bovine leukosis virus producing cell line" JOURNAL OF VIROLOGICAL METHODS, ELSEVIER BV, NL, vol. 121, no. 2, 1 November 2004 (2004-11-01), pages 239-246, XP004572269 ISSN: 0166-0934
- RUSSO S ET AL: "Expression of bovine leukemia virus ENV glycoprotein in insect cells by recombinant baculovirus", FEBS LETTERS, ELSEVIER, AMSTERDAM, NL, vol. 436, no. 1, 25 September 1998 (1998-09-25), pages 11-16, XP004258350, ISSN: 0014-5793, DOI: 10.1016/S0014-5793(98)00951-X

## Description

The subject of the present invention is a method of obtaining purified BLV gp51 antigen. A novel ELISA assay using said antigen is also described. The present invention is useful in the diagnosis of enzootic leukaemia in cattle.

Enzootic bovine leukaemia (EBL) is an infectious disease of cattle caused by a type C retrovirus, BLV (Bovine Leukaemia Virus) and consists of lymphatic node hypertrophy. The disease is characterised by a long incubation period (up to 7 years) and, in most cases, (ca. 60%) proceeds without symptoms. Lymphatic cysts form in a portion of adult cattle (10-30%), whereas 1-10% develop lymphatic sarcomas on various internal organs, with a marked increase of B lymphocytes.

A strong immune response occurs in infected individuals, which is used in serological diagnostics. Despite the fact that the titre of anti-BLV antibodies increases as the disease progresses, they are not able to halt the infection. Because the disease develops asymptomatically through a long initial phase, the only effective method of preventing transmission are frequent serological diagnostics and the elimination of infected animals. Serological assays are performed on animals over 6 months, when maternal antibodies have begun disappearing. The virus itself can be detected in isolated peripheral lymphocytes using an electron microscope, and viral DNA can be diagnosed using PCR. Immunological assays are used most frequently to diagnose bovine leukaemia: gel immunodiffusion (AGID), immunoenzymatic assays (ELISA) as well as radioimmunological detection. These methods make use of antibodies against the antigenic proteins gp51 and p24.

ELISA assays for diagnosing enzootic bovine leukaemia are based on the determination of anti-BLV antibody levels in serum or milk. To construct the assay kit it is necessary to produce purified gp51 viral surface antigen. This antigen is produced in a culture of FLK cells infected with BLV. Cell cultures make frequent use of calf serum (FCS) containing bovine immunoglobulins that, despite tedious purification procedures, contaminate the resulting preparations with bovine antibodies, which leads to false positive results.

Available literature describes a series of labour-intensive methods of purifying the gp51 antigen, encompassing precipitation, extraction, centrifugation in a saccharose gradient, gel and ion exchange chromatography (Grunboeck M. et al., Polskie Archiwum Weterynaryjne 1986,24, 327-336). Ukrainian patent (UA 68930 A) describes a culture medium containing avian serum (instead of bovine). De Guiseppe et al. (Clinical and Diagnostic Laboratory Immunology, 2004, vol 11, no.1, p.147-151) disclose an ELISA using baculovirus expressed BLV gp51. An ELISA using immuno-affnity purified BLV gp51 produced in FLK-BHV cells is shown in Merza et al. (Journal of Virological Methods, 1991, vol.33, p.345-353). The production of the purified antigen, however, requires the removal of a large quantify of ballasting avian antigens.

The unsolved problem in prior art are the difficult, tedious and expensive methods of purifying the antigen to be used in the ELISA assay. At the same time, due to the problems at this stage, contaminated antigen preparations are produced, which, when used in an ELISA assay, lead to erroneous results and make rapid and effective diagnostics of the disease impossible.

The subject of the present invention is a method of obtaining pure BLV gp51 antigen characterised in that the antigen production process uses culture media totally devoid of bovine serum as defined in the claims.

Culture media for FLK-BLV cells are used.

In an embodiment of the present invention, the culture media encompass HyQ SFM4MegaVir, HyQ PF-Vero, and Gibco Opti Pro SFM.

Purified BLV gp51 antigen may be used in a gel immunodiffusion assay or an ELISA assay.

A novel ELISA assay for detecting enzootic leukaemia in cattle is described, characterised in that it contains a highly pure BLV gp51 BLV antigen obtained from cell culture media totally devoid of bovine serum.

The present invention, is described below.

### Example

FLK-BLV cells are cultured in dishes or culture flasks in DMEM medium without bovine serum, for example HyQ SFM4MegaVir, HyQ PF-Vero and Gibco Opti Pro SFM. The culture is maintained for 2-3 days until the cells reach a large density, and then for the subsequent several days, the medium is collected from above the cells and is maintained at -20°C. The collected medium is condensed 10-fold via ultrafiltration using an Amicon YM10 membrane and dialysed against 20 mM Tris-HCl pH 7.5, whereafter it is purified in a DEAE-Sepharose FF column equilibrated with 20 mM Tris-HCl pH 7.5 and eluted with a sodium chloride gradient (0-500 mM). Antigen gp51-containing fractions are pooled, dialysed against PBS and stored at -20 C.

The antigen thus obtained can be used in gel immunodiffusion assays or ELISA assays.

## Claims

1. A method of producing purified BLV gp51 antigen that is useful in an ELISA assay for diagnosing enzootic bovine leukaemia using FLK-BLV cells, comprising;
a) cultivation of FLK-BLV cells in DMEM medium that is devoid of bovine serum, wherein the culture of FLK-BLV cells is maintained for 2 to 3 days until the cells reach a large density, and then
b) collecting the medium for several subsequent days from the FLK-BLV cells containing the BLV gp51 antigen produced by the FLK-BLV cells;
c) condensing the medium at least 10-fold by ultrafiltration using Amicon YM10 membrane having a nominal molecular weight limit of 10 KDa;
d) dialyzing the condensed medium against 20mM Tris-HCl pH 7.5; and purifying the BLV gp51 antigen in a DEAE-Sepharose fast flow (FF) column, wherein the BLV gp51 antigen is eluted from the column with sodium chloride gradient of 0-500 mM.

2. A method according to Claim 1, **characterised in that** the medium collected in step b) is maintained at -20°C prior to performing step c).

## Patentansprüche

1. Ein Verfahren zur Herstellung gereinigten BLV-gp51-Antigens, das in einem ELISA-Assay zur Diagnose enzootischer boviner Leukämie mithilfe von FLK-BLV-Zellen hilfreich ist, umfassend:
a) Kultivieren von FLK-BLV-Zellen in DMEM-Medium, welches von bovinem Serum frei ist, wobei die Kultur von FLK-BLV-Zellen über 2 bis 3 Tage gepflegt wird, bis die Zellen eine hohe Dichte erreichen, und dann
b) Ernten des Mediums von den FLK-BLV-Zellen, das das von den FLK-BLV-Zellen hergestellte BLV-gp51-Antigen enthält, über mehrere folgende Tage;
c) mindestens 10faches Einengen des Mediums durch Ultrafiltration mithilfe einer Amicon-YM10-Membran mit einer nominalen Molekulargewichtsgrenze von 10 kDa;
d) Dialysieren des eingeengten Mediums gegen 20 mM Tris-HCl pH 7.5; und Reinigen des BLV-gp51-Antigens über eine DEAE-Sepharose-Schnelldurchfluss(FF)-Säule, wobei das BLV-gp51-Antigen mit einem Natriumchloridgradienten von 0-500 mM eluiert von der Säule wird.

2. Ein Verfahren nach Anspruch 1, dadurch charakterisiert, das das in Schritt b) geerntete Medium vor der Durchführung des Schrittes c) bei -20 °C gehalten wird.

## Revendications

1. Procédé de production d'un antigène gp51 de BLV purifié qui est utile dans un test ELISA pour diagnostiquer la leucémie bovine enzootique en utilisant des cellules FLK-BLV, comprenant:
a) la culture de cellules FLK-BLV dans du milieu DMEM qui est dépourvu de sérum bovin, dans laquelle la culture de cellules FLK-BLV est maintenue pendant 2 à 3 jours jusqu'à ce que les cellules atteignent une grande densité, puis
b) la collecte du milieu, pendant plusieurs jours consécutifs, des cellules FLK-BLV, contenant l'antigène gp51 de BLV produit par les cellules FLK-BLV;
c) la concentration du milieu au moins par un facteur 10 par ultrafiltration en utilisant une membrane Amicon YM10 ayant une limite de poids moléculaire nominal de 10 kDa;
d) la dialyse du milieu concentré contre du Tris-HCl 20mM pH 7,5 ; et la purification de l'antigène gp51 de BLV dans une colonne DEAE-Sepharose fast flow (FF), l'antigène gp51 de BLV étant élué de la colonne avec un gradient de chlorure de sodium de 0 à 500 mM.

2. Procédé selon la revendication 1, **caractérisé en ce que** le milieu collecté à l'étape b) est maintenu à -20°C avant d'effectuer l'étape c).
